# EUROPEAN PATENT SPECIFICATION

(11) **EP 1 729 788 B1**
(45) Date of publication and mention of the grant of the patent: **25.11.2009**
(21) Application number: 05727807.9
(22) Date of filing: 29.03.2005
(51) Int. Cl.: A61K 35/28, A61P 9/10

(54) **RESTENOSIS THERAPY USING MESENCHYMAL STEM CELLS**
RESTENOSE-THERAPIE MIT MESENCHYM-STAMMZELLEN
TRAITEMENT DE LA RESTENOSE A L'AIDE DE CELLULES SOUCHES MESENCHYMATEUSES

(30) Priority: 30.03.2004 US 557388 P
(43) Date of publication of application: 13.12.2006
(73) Proprietor: Boston Scientific Limited, St. Michael (BB)
(72) Inventor: FREYMAN, Toby, Waltham, MA 02453 (US)
(74) Representative: Jungblut & Seuss
(86) International application number: PCT/US2005/010146
(87) International publication number: WO 2005/097147

(56) References cited:
- WO-A-00/06701
- WO-A-03/073998
- TANG Y L ET AL: "Autologous mesenchymal stem cell transplantation induce VEGF and neovascularization in ischemic myocardium" REGULATORY PEPTIDES 15 JAN 2004 NETHERLANDS, vol. 117, no. 1, 15 January 2004 (2004-01-15), pages 3-10, XP002333008 ISSN: 0167-0115
- WALTERS DARREN L ET AL: "Acute coronary syndrome is a common clinical presentation of in-stent restenosis" AMERICAN JOURNAL OF CARDIOLOGY, vol. 89, no. 5, 1 March 2002 (2002-03-01), pages 491-494, XP002333023 ISSN: 0002-9149
- WERNER NIKOS ET AL: "Intravenous transfusion of endothelial progenitor cells reduces neointima formation after vascular injury." CIRCULATION RESEARCH. 25 JUL 2003, vol. 93, no. 2, 25 July 2003 (2003-07-25), pages e17-e24, XP002333009 ISSN: 1524-4571

## Description

### Technical Field

The present invention relates to methods for alleviating the negative effects of vascular trauma, and in particular to treating restenosis following vascular surgery.

### Background of the Invention

Heart disease is the No. 1 killer of American men and women, affecting more than 12 million Americans. Coronary Artery Disease (CAD) is atherosclerosis of the arteries that provide vital oxygen and nutrients to the heart. Atherosclerosis occurs when the arteries become clogged and narrowed, restricting blood flow to the heart. Without adequate blood, the heart becomes starved of oxygen and vital nutrients it needs to work properly.

Atherosclerosis (for review see Ross, R. Nature 362:801-809 (1993) and Hajjar et al., Amer. Scientist 83:460-467(1995)) is the principal cause of heart attacks, stroke, gangrene and loss of function of extremities. It accounts for approximately 50% of all mortalities in the USA, Europe and Japan (Ross, R. (1993) Nature 362:801-809). The present therapeutic strategies for severe atherosclerosis in coronary arteries rely on angioplasty procedures (e.g., percutaneous trans-luminal coronary angioplasty (PTCA), directional coronary atherectomy (DCA) or related angioplasty procedures), and coronary artery bypass surgery. During angioplasty, a specially designed catheter with a small balloon tip is guided to the point of narrowing in the artery. Once in place, the balloon is inflated to compress the plaque into the artery wall and stretch the artery open to increase blood flow to the heart. For example, PTCA is the primary treatment modality in many patients with coronary heart disease. PTCA can relieve myocardial ischemia in patients with coronary artery disease by reducing lumen obstruction and improving coronary bloodflow. In addition to angioplasty, a number of coronary interventions, such as coronary stenting, cutting balloons, and rotoblators, have been used in the treatment of CAD.

Balloon dilation of moderately or clinically significant stenoses or occlusions fractures the arterial plaque, separates the plaque from the underlying artery wall and stretches the artery wall, to create or enlarge the artery lumen. At the time of angioplasty, the dilated balloon may injure and/or make tears in the atherosclerotic plaques, subsequently forming a sufficiently large luminal opening. As a result, intimal hyperplasia, which originates from the sites of injured plaques, can occur several months after angioplasty. Intimal hyperplasia may play a central role in restenosis after angioplasty.

While the use of interventional procedures has grown rapidly, reocclusion (or restenosis) of arteries is a serious complication which occurs in 30-50% of patients undergoing various angioplasty procedures within 3 days to 3 months. Restenosis results in significant morbidity and mortality and frequently necessitates further interventions, such as repeat angioplasty or coronary bypass surgery.

Since the introduction of coronary angioplasty in 1977, the problems of abrupt vessel closure and restenosis have become increasingly conspicuous and relatively intractable with balloon technology. The concept of endoluminal stents was first introduced in 1978, and these devices have been used to manage the problems of abrupt vessel closure and restenosis with varying results. Some investigators have reported a reduction in restenosis rates but others have found no significant effect. Willerson, Circulation 96:383-385 (1997).

A stent is a small stainless steel mesh tube that acts as a scaffold to provide support inside the coronary artery. A balloon catheter is used to insert the stent into the narrowed coronary artery. When the balloon tip is inflated, the stent expands to the size of the artery. The balloon is deflated and removed, and the stent stays in place permanently. Abrupt vessel closure has been successfully treated with intracoronary stents. However, acute and subacute thrombotic occlusions of stented vessels remain a significant problem.

Both angioplasty and stent implantation produce a local injury to the wall of the blood vessel and often lead to subsequent re-occlusion (restenosis) of the blood vessel. Although the processes responsible for restenosis are not completely understood, restenosis has been suggested to occur, at least in part, as a result of local inflammation, thrombosis and smooth muscle cell migration and proliferation within the intima of coronary arteries. To date, no post-angioplasty treatment has proven effective in the prevention or treatment of restenosis.

Thus, there is a need for methods and compositions for preventing and/or treating restenosis. Preferably, these methods and compositions are specific in their effect, easy to administer, and effective with minimal adverse side-effects.

### Summary of the Invention

In accordance with the present invention, mesenchymal stem cells are used to prevent restenosis of blood vessels that have sustained injury, especially as a result of PTCA or stenting.

Accordingly, one aspect of the invention provides a medical use for reducing restenosis of a blood vessel, comprising administering a therapeutic amount of mesenchymal stem cells to a patient at risk for developing restenosis as a result of experiencing a vascular trauma caused by a surgical procedure. Surgical procedures that may cause vascular trauma include, *inter alia,* angioplasty, stent implantation, rotoblation, atheroectomy, thrombectomy, and grafting. Preferably the therapeutic amount of mesenchymal stem cells will be administered to the patient concurrent with or up to 14 days after infliction of the vascular trauma. Most preferably the mesenchymal stem cells are administered within 3 days of the trauma.

The mesenchymal stem cells that are employed can be a homogeneous composition or can be a mixed cell population enriched in MSCs. Homogeneous human mesenchymal stem cell compositions can be obtained by culturing adherent marrow or periosteal cells. Methods for isolating and expanding populations of mesenchymal stem cells are described below in detail.

Another aspect of the invention is the reduction of scar formation as a result of angioplasty, by administering a therapeutic amount of mesenchymal stem cells to an angioplasty patient. Yet another aspect of the invention is enhancing the repair of vascular damage by administering a therapeutic amount of mesenchymal stem cells to a patient having vascular damage. Because the vascular tissue that is damaged in the process of performing an angioplasty is in contact with circulating blood, the mesenchymal stem cells are preferably administered intravenously. The mesenchymal stem cells can be from a spectrum of source including, in order of preference, autologous, allogenic or xenogenic.

### Definitions

The term "restenosis" refers to a recurrence of stenosis. The term "stenosis" as used herein refers to a narrowing of any canal in the circulatory system including, but not limited to, valves (e.g., aortic stenosis which involves narrowing of the aortic valve orifice), coronary arteries (e.g., coronary ostial sclerosis which involves narrowing of the mouths of the coronary arteries), carotid arteries, renal arteries, etc. Restenosis generally results from neointimal hyperplasia. The term "neointimal hyperplasia" refers to the development of a proliferative lesion in the intimal layer of a blood vessel. Neointimal hyperplasia results, for example, from migration of smooth muscle cells of the tunica media layer of the blood vessel toward the lumen into the subintimal space below the endothelium (i.e., the inner lining of the blood vessel). These smooth muscle cells proliferate within the intimal space and create a "mass effect" that narrows the vessel lumen and reduces oxygenation and nutritive blood flow.

As used herein, the term "therapeutic amount" refers an amount mesenchymal stem cells, measured by the number of cells, required to reduce, delay, or eliminate undesirable pathologic effects in a subject. A "therapeutic amount" can be any amount greater than the minimal therapeutic amount up to, but excluding, the amount at which the negative effects of administration of mesenchymal stem cells outweigh the benefits of such administration. A "therapeutic amount" of mesenchymal stem cells, when made in reference to restenosis, refers to that amount of the compound which would diminish restenosis. A "minimum therapeutic amount" is the fewest number of mesenchymal stem cells required to reduce, delay, or eliminate restenosis in a subject.

The term to "diminish restenosis," as used herein in reference to the effect of a particular composition or of a particular method is meant to reduce, delay, or eliminate restenosis as compared to the level of restenosis observed in the absence of treatment with the particular composition or method. As used herein, the term "reducing" restenosis refers to decreasing the intimal thickening that results from stimulation of smooth muscle cell proliferation. The term "delaying" restenosis refers to increasing the time period between removal of a stenosis (e.g., by use of surgical procedures) and onset of visible intimal hyperplasia (e.g., observed histologically or by angiographic examination). The term "eliminating" restenosis refers to completely "reducing" intimal thickening and/or completely "delaying" intimal hyperplasia in a subject to an extent which makes it no longer necessary to surgically intervene in order to re-establish a suitable blood flow through the vessel by surgical means (e.g., by repeating angioplasty, atherectomy, or coronary artery bypass surgery). The effects of diminishing restenosis in a human subject can be determined by methods routine to those skilled in the art including, but not limited to, angiography, ultrasonic evaluation, fluoroscopic imaging, fiber optic endoscopic examination or biopsy and histology. The effects of diminishing restenosis in a non-human animal subject can be determined by, for example, methods described herein including a reduction in the intimal/media cross-sectional ratio as measured by light microscopy of formalin-fixed tissue.

The term "atherosclerosis" refers to a form of arteriosclerosis in which deposits of yellowish plaques (i.e., atheromas) containing cholesterol, lipoid material, and lipophages are formed within the intima and inner media of large and medium-sized arteries.

The term "angioplasty" refers to surgery of blood vessels as exemplified by percutaneous transluminal coronary angioplasty (PTCA), wherein a balloon in a catheter is inflated to open the lumen of an artery blocked by atherosclerotic plaques to allow blood flow, and by directional coronary atherectomy (DCA), wherein an atherosclerotic plaque is removed from the lumen of a blocked artery.

### Detailed Description of the Invention

Mesenchymal stem cells (MSC) are pluripotent progenitors for a variety of cell types of mesenchymal cell lineage, including bone, cartilage, fat, tendon, nerve tissue, fibroblasts and muscle cells. Mesenchymal stem cells can be isolated and purified from tissue such as bone marrow, blood (including peripheral blood), periosteum and dermis, and other tissues which have mesodermal origins. In this regard, it has been found that although these progenitor mesenchymal stem cells are normally present in bone marrow, for example, in very minute amounts and that these amounts greatly decrease with age (i.e. from about 1/10,000 cells in a relatively young patient to as few as 1/2,000,000 in an elderly patient), human mesenchymal stem cells can be isolated from tissue and purified when cultured in a specific medium by their selective attachment, termed "adherence," to substrates.

The human mesenchymal stem cells can be derived, for example, from bone marrow obtained from a number of different sources, including plugs of femoral head cancellous bone pieces, obtained from patients with degenerative joint disease during hip or knee replacement surgery, and from aspirated marrow obtained from normal donors and oncology patients who have marrow harvested for future bone marrow transplantation. Although the harvested marrow is generally prepared for cell culture separation by a number of different mechanical isolation processes depending upon the source of the harvested marrow (i.e. the presence of bone chips, peripheral blood, etc.), the critical step involved in the isolation processes is the use of a specially prepared medium that contains agents that allow for not only mesenchymal stem cell growth without differentiation, but also for the direct adherence of only the mesenchymal stem cells to the plastic or glass surface area of the culture dish. By producing a medium that allows for the selective attachment of the desired mesenchymal stem cells, which are present in the marrow samples in very minute amounts, it is possible to separate the mesenchymal stem cells from the other cells (i.e. red and white blood cells, other differentiated mesenchymal cells, etc.) present in the bone marrow.

While the invention is not limited to the use of mesenchymal stem cells obtained by any particular method, MSC can be obtained from bone marrow, purified and culturally-expanded as follows. Plugs or aspirates of bone marrow are processed to remove bone pieces and fat. The marrow cells (consisting predominantly of red and white blood cells, and a very minute amount of mesenchymal stem cells) are passed through syringes to dissociate the tissue into single cells. The cells are cultured in complete medium (e.g., BGI_{b} medium with 10% fetal bovine serum) and humidified atmosphere. The media is not changed for at least one day to allow the cells to attach to the culture dish. Thereafter the media is replaced every 3-4 days. When the cells have grown to confluence, the cells are detached from the culture dish, preferably with trypsin. Cells can be subcultured in serum-free media after removal or inactivation of the trypsin. Mesenchymal stem cells will not express markers that identify differentiated mesenchymal phenotypes such as type II collagen (chondrocytes), Bone Gia Protein (BGP) (osteoblasts), laminin, elastin and type IV collagen (basement membrane fibroblasts), Stro-1 antigen (marrow stromal cells), or von Wildebrand factor (endothelial cells). MSCs also do not express the typical hematopoietic antigens, CD45, CD34, and CD14. In contrast, MSCs express high levels of IL-6.

Lineage development of MSC is influenced by their surrounding microenvironment. In vitro experiments have demonstrated that culture conditions, additives, growth factors and cytokines can precisely induce MSC to develop into a selected mesenchymal tissue. For example, dexamethasone in combination with isobutilmethylxanthine or insulin or a mixture of isobutilmethylxanthine, insulin and indomethacin have been shown to push the MSCs toward differentiating into adipocytes. Similarly, MSCs can differentiate into skeletal muscle cells when stimulated with 5-azacytidine. β-FGF has been shown to cause mesenchymal stem cells to differentiate into cardiac muscle cells. It is believed that local environments within the body will also influence the differentiation of mesenchymal stem cells *in vivo.*

Thus, the isolated and purified human mesenchymal stem cells can be grown in an undifferentiated state through mitotic expansion in a specific medium. These cells can then be harvested and activated to differentiate into bone, cartilage, and various other tissues of mesenchymal lineage by a number of factors, including mechanical, cellular, and biochemical stimuli. It has been determined that human mesenchymal stem cells possess the potential to differentiate into cells such as endothelial cells or osteoblasts and chondrocytes, which produce a wide variety of mesenchymal tissue cells, as well as tendon, ligament and dermis, and that this potential is retained after isolation and for several population expansions in culture.

The general sequence of events that are activated in response to injury and that lead to successful wound healing comprise (1) the activation of the coagulation system, leading to a cessation of blood flow and the formation of a provisional matrix; (2) the local generation of a variety of soluble chemotactic factors formed from preformed plasma proteins that attract inflammatory cells to the site of injury; (3) the sequential influx of neutrophils and monocytes, leading to wound sterilization; (4) the debridement of damaged connective tissue matrix; (5) the initiation of neovascularization; and (6) the stimulation of mesenchymal cell proliferation and connective tissue matrix remodeling. While in many tissues and situations, this generalized sequence of events leads to the restoration of normal tissue structure and functions, in some tissues repair is invariably associated with scarring. Without being bound to a particular theory, the administered mesenchymal stem cells are believed to accelerate healing and decrease scarring by secreting factors, such as TGF-β, that suppress the inflammatory response. MSCs have been used in other situations to promote healing, e.g., of bone. However, they have not previously been used to avoid restenosis associated with vascular damage.

In accordance with the present invention, MSCs are infused into the circulatory system of a patient, wherein at least a portion of the administered cells home to and populate the damaged vascular tissue. Further, signals originating in the vascular tissue appear to modify the developmental program of the infused MSCs, accelerating the wound healing process.

The present invention provides methods for the treatment of restenosis. In one embodiment, the methods of the invention comprise administering a therapeutic amount of mesenchymal stem cells to a patient at risk for developing restenosis under conditions such that restenosis symptoms are diminished. In a preferred embodiment, the restenosis sought to be alleviated by the methods of the invention is that which may follow vascular trauma from vascular surgical procedures such as angioplasty. Angioplasty can be performed, for example, by percutaneous trans-luminal coronary angioplasty (PTCA) or by directional coronary atherectomy (DCA). PTCA generally involves inserting a catheter (i.e., a plastic tube) with a balloon on the end into the blood vessel and inflating the balloon to high pressures to dilate the lumen of a blood vessel that is narrowed e.g. by atherosclerosis (i.e., hardening of the artery). DCA involves inserting a catheter with a probe at the end (the probe is generally metallic in order to permit X-ray visualization during the surgical procedure) into the blood vessel and removing atherosclerotic tissue from the lumen of the vessel with the probe.

However, the methods of the invention are not limited to vascular trauma from angioplastic procedures. Any procedure which results in restenosis is contemplated to be within the scope of the invention. Such procedures include, for example, rotoblation, atheroectomy, placement of a stent (e.g., in a vessel), thrombectomy, and grafting. Atheroectomy can be performed, for example, by surgical excision, ultrasound or laser treatment, or by high pressure fluid flow. Introduction of a stent generally involves introducing a wire-mesh cylinder within the lumen of a stenotic vessel to increase the lumen diameter and restore blood flow. Thrombectomy can be performed by, for example, introducing into the vessel a pneumatically operated catheter which is fitted with rotating blades at the tip to remove the thrombus or clot. Grafting can be achieved, for example, by vascular grafting using natural or synthetic materials, or by surgical anastomosis of vessels such as during organ grafting.

The mesenchymal stem cells used in accordance with the invention are, in order of preference, autologous, allogeneic or xenogeneic, and the choice can largely depend on the urgency of the need for treatment.

Those skilled in the art will recognize that initial indications of the appropriate therapeutic dosage of the mesenchymal stem cells for a given vascular surgical procedure can be determined in *in vitro* and *in vivo* animal model systems, and in human clinical trials. *In vitro* testing for a particular cell type or lineage can be accomplished using commercially available smooth muscle or endothelial cells coupled with determination of the effect of mesenchymal stem cell treatment on wound healing as measured by Western blot analysis, cellular proliferation and migration, and on extracellular matrix invasion.

MSCs can be marked in a variety of ways prior to their introduction into the recipient. This makes it possible to trace the fate of the MSCs as they proliferate and differentiate in the weeks following the MSC implant. Several methods are utilized to positively identify the injected cells: membrane lipid dyes PKH26 or CM-DI I and genetic marking with adeno-associated virus (AAV) or retroviruses, such as Maloney murine leukemia virus expressing green fluorescent protein (GFP) or galactosidase. Alternatively, particles, typically < 1µm in diameter, may be exposed to and internalized by the MSCs. The particles typically are composed of iron oxide, gold or iridium. The internalized particles can be detected by a variety of methods including MRI, histology or neuron activity. PCR may also be used to detect the Y chromosome marker of male cells implanted into female animals. Dye-labeled cells are readily detected and offer the simplest method to directly follow the injected cells. This method is reliable for times out to at least 4 weeks. On the day of introduction to recipient animals, MSCs are trypsinized and labeled with CM-DI I according to the recommendations of the manufacturer (Molecular Probes). Subconfluent monolayer cultures of MSCs are incubated with 5 mM CM-DI I in serum-free medium for 20 minutes, trypsinized, washed twice in excess dye-free medium, and utilized for injection.

Alternatively, MSCs are genetically marked prior to use, such as by using AAV-GFP vector. This vector lacks a selectable marker but mediates high-level expression of the transduced genes in a variety of post-mitotic and stem cell types. Recombinant AAV-GFP is added to low density monolayers of MSCs in low serum. Following a four hour incubation at 37°C, the supernatant is removed and replaced with fresh media. At 96 hours after transduction, cells are assayed for green fluorescent protein (GFP) activity. Typically 50% of the cells express the transduced gene. Unselected MSCs on a clonal line, isolated by limiting dilution, are utilized for injection. Cells are collected following trypsin treatment, washed and used at high concentrations for testing and/or treatment (10 to 100 million cells per ml).

*In vivo* determination of a suitable therapeutic dose can be accomplished using art-accepted animal models such as the rat carotid artery model described herein, in which the effect of mesenchymal stem cell treatment on DNA synthesis and intimal/medial cross-sectional ratios are determined. However, as the present situation involves treatment under the care of a physician, adjustments will be made to the amount and timing of treatment during the course of treatment, based on the physician's evaluation of the effectiveness of the treatment, which will vary from patient to patient.

As a representative example, a dose range is a volume of about 1 to about 50 ml of injectible suspension containing up to 2 x 10⁷ MSCs/ml. Preferably, 10,000 to 3 million MSC/kg are administered in a single injection. Preferably, the mesenchymal stem cells are administered intravenously. The most preferred method of administration is via intravenous injection. The injection medium can be any pharmaceutically acceptable isotonic liquid. Examples include phosphate buffered saline (PBS), culture media such as DMEM (preferably serum-free), physiological saline or 5% dextrose in water. Optionally, the medium may contain up to 10% DMSO.

The timing and frequency of treatment can vary. Preferably, the mesenchymal stem cells will be administered in conjunction with an inflammatory response at the site of vascular trauma. However, the mesenchymal stem cells may be administered to the patient as early as one week before the vascular trauma and as late as 2 weeks after incidence of the trauma. Preferably, the mesenchymal stem cells will be administered at anytime from immediately after the occurrence of the vascular trauma until about 14 days after the trauma. More preferably, the cells will be administered within about 7 days after occurrence of the vascular trauma. Most preferably, the mesenchymal stem cells will be administered within about 3 days after occurrence of the vascular trauma. Continuous or multiple doses may be administered during this time window. The continuous or multiple doses may be accomplished through catheters or implanted pumps, e.g., an osmotic pump.

A conventional syringe or a controllable arthroscopic delivery device can be used so long as the needle lumen or bore is of sufficient diameter (e.g. 30 gauge or larger) that shear forces will not damage the MSCs. The injectible liquid suspension MSC preparations can also be administered intravenously, either by continuous drip or as a bolus.

Solutions of mesenchymal stem cells can be prepared in suitable isotonic liquids such as phosphate buffered saline, culture media, such as DMEM, physiological saline, 5% aqueous dextrose, and/or mixtures thereof, and other suitable liquids known to those skilled in the art. The final therapeutic form should be protected against contamination and should, therefore, be able to inhibit the growth of microorganisms such as bacteria or fungi. A single intravenous dose can be administered. Alternatively, a slow long term infusion or multiple short term daily infusions can be utilized, typically lasting from 1 to 8 days. Alternate day or dosing once every several days can also be utilized.

Prevention or inhibition of growth of microorganisms can be achieved through the addition of one or more antimicrobial agents such as chlorobutanol, ascorbic acid, parabens, thermerosal, or the like. It may also be preferable to include agents that alter the tonicity such as sugars or salts.

The present invention can be practiced, but is not limited, in the manner shown by the following examples.

### Example 1

The first example shows how initial dose levels can be determined using a rat model. Rat MSCs are introduced by direct injection into athymic rats. To analyze the injected cells over several weeks and to minimize the possibility of immune system rejection, MSCs are harvested from the same inbred strain (identical genotype) as the intended MSC recipients.

A suspension of labeled MSC is directly injected into carotid traumatized rats using a 28 gauge needle. In order to traumatize rat arteries, male Sprague-Dawley rats (average weight 500 g) (Charles Rivers) are anaesthetized with Nembutal (4 mg per 100 g), and the left carotid artery of each animal is isolated by a midline cervical incision, suspended on ties and stripped of adventitia. A 2F Fogarty catheter is introduced through the external carotid artery of each rat, advanced to the aortic arch, the balloon is inflated to produce moderate resistance to catheter movement and then gradually withdrawn to the entry point. The entire procedure is repeated three times for each animal. After vascular injury to the carotid artery, the distal injured segment is transiently isolated by temporary ligatures.

At different time intervals ranging from 2 days to 4 weeks following the injection, the rats are sacrificed and the location of the vascular damage is studied histologically. When sacrificed, the carotid artery is removed, examined by light microscopy for the presence of vascular thrombi or emboli, paraffin-embedded, and sectioned. The histology of serial sections is examined to determine the fate of dye-stained cells. The integration of implanted cells, their subsequent differentiation, formation of junctions with vascular endothelial cells, and their long-term survival are characterized with light microscopy, histology, confocal immunofluorescence microscopy, electron microscopy and in situ hybridization. Sections are tested for immunohistochemical markers of vascular endothelial tissue in the areas of the introduced MSCs to ascertain whether donor MSCs have differentiated in vivo.

### Example 2

This example demonstrates an *in vivo* method of determining an initial therapeutic dose in rats. Traumatization of the rat carotid artery is an art-accepted method for investigating restenosis. Lee et al. Circulation Research 73:797-807 (1993); von der Leyen et al. FASEB J. 8:A802 (1994); Simons et al. Nature 359:67-70 (1992); Edelman et al. J. Clin,. Invest. 89:465-473 (1992); Morishita et al. Proc. Natl. Acad. Sci. USA 90:8474-8478 (1993).

In order to traumatize rat arteries, the adventitia of the carotid artery are stripped as previously described (Simons et al. (1992) Nature 359:67-70 (1992); Edelman et al. (1992) J. Clin,. Invest. 89:465-473; Morishita et al. (1993) Proc. Natl. Acad. Sci. USA 90:8474-8478]) by subjecting the left common carotid arteries of rats to balloon angioplasty which denudes endothelium and induces a highly reproducible intimal migration/proliferation of SMCs over the entire length of the affected blood vessel. Briefly, male Sprague-Dawley rats (average weight 500 g) (Charles Rivers) are anaesthetized with Nembutal (4 mg per 100 g), and the left carotid artery of each animal is isolated by a midline cervical incision, suspended on ties and stripped of adventitia. A 2F Fogarty catheter is introduced through the external carotid artery of each rat, advanced to the aortic arch, the balloon is inflated to produce moderate resistance to catheter movement and then gradually withdrawn to the entry point. The entire procedure is repeated three times for each animal. After vascular injury to the carotid artery, the distal injured segment is transiently isolated by temporary ligatures.

The rats are divided into 6 groups. Group 1 receive 1x10⁶ mesenchymal stem cells suspended in 50 µl phosphate buffered saline by intravenous injection immediately after traumatization of the carotid artery. Rats in untreated Group 2 are intravenously injected with 50 µl phosphate buffered saline immediately after traumatization of the carotid artery. Rats in Group 3 receive 1x10⁶ mesenchymal stem cells suspended in 50 µl phosphate buffered saline by intravenous injection 24 hours after traumatization of the carotid artery. Rats in untreated Group 4 are intravenously injected with 50 µl phosphate buffered saline 24 hours after traumatization of the carotid artery. Rats in Group 5 receive 1x10⁶ mesenchymal stem cells suspended in 50 µl phosphate buffered saline by intravenous injection 5 days after traumatization of the carotid artery. Rats in untreated Group 6 are intravenously injected with 50 µl phosphate buffered saline 5 days after traumatization of the carotid artery.

The effect of administration of mesenchymal stem cells on restenosis is determined by measurement of vascular DNA synthesis and content, and of the effect on neointimal size. For bromodeoxyuridine (BrdUrd) staining, BrdUrd is injected into rats after vascular injury (100 mg/kg subcutaneously and 30 mg/kg intraperitoneally at 18 h prior to sacrifice and then 30 mg/kg intraperitoneally at 12 h prior to sacrifice). Rats are sacrificed on day 7 after traumatization of the carotid artery. The carotid artery is removed after perfusion-fixation (110 mmHg; 1 mmHg=133 Pa) with 4% (wt/vol) paraformaldehyde and processed for immunohistochemistry by using anti-BrdUrd antibodies (Amersham). The proportion of BrdUrd-positive cells is determined by cell counts under light microscopy in a blinded fashion. Measurement of DNA is performed at 7 days after traumatization of the carotid artery using bisbenzimide trihydrochloride (Pierce). It is expected that mesenchymal stem cell treatment of injured arteries will inhibit BrdUrd incorporation (a marker of DNA synthesis and cell proliferation) in the vessel wall as compared to the untreated injured control vessels.

Formation of neointima along the length of the treated artery is determined at 2, 4, and 8 weeks after the surgical procedure. At the time of killing, the animals are anaesthetized with Nembutal and perfused with 150 cc normal saline under a pressure of 120 mm Hg. The carotid arteries are removed, fixed in 3% formalin, and processed for light microscopy in a standard manner. Three individual sections from the middle of surgically treated segments from each rat are analyzed by measuring the mean cross-sectional areas of the intimal and of the medial regions which are untreated, treated with carrier alone or treated with mesenchymal stem cells. These measurements are used to determine a ratio of intimal to medial cross-sectional areas. Animals are coded so that operation and analysis are performed without knowledge of which treatment individual animals receive. It is expected that treatment with mesenchymal stem cells will result in a reduction of the cross-sectional ratio of intima/media as compared with the cross-sectional ratio of intima/media in control injured arteries receiving no treatment. Such a reduction in ratio indicates that the mesenchymal stem cells are useful in reducing restenosis in a human subject.

### Example 3

This example demonstrates treatment with mesenchymal stem cells of a human having vascular trauma. An angioplasty patient receives 500,000 mesenchymal stem cells suspended in 20 ml phosphate buffered saline by intravenous injection immediately after completion of the angioplasty. Volumetric blood flow is measured by simultaneously assessing vessel size (using either quantitative angiography or intravascular ultrasound) and blood flow velocity (using intravascular Doppler or measuring TIMI flow through the treated section).

Although the invention has been described in connection with specific preferred embodiments, it should be understood that the invention as claimed should not be unduly limited to such specific embodiments. Indeed, various modifications of the described modes for carrying out the invention which are obvious to those skilled in the art are intended to be within the scope of the following claims.

## Claims

1. Use of mesenchymal stem cells excluding human embryonic mesenchymal stem cells in the manufacture of a medicament for reducing restenosis of a blood vessel in a patient that has experienced a vascular trauma; wherein the vascular trauma is caused by a surgical procedure, e.g. selected from the group consisting of angioplasty, stent implantation, rotoblation, atheroectomy, thrombectomy, and grafting.

2. The use of claim 1, wherein (i) a therapeutic amount of mesenchymal stem cells is administered in conjunction with an inflammatory response at the site of vascular trauma; or (ii) a therapeutic amount of mesenchymal stem cells is administered between immediately after occurrence of the vascular trauma and about 14 days after occurrence of the vascular trauma; optionally wherein the therapeutic amount of mesenchymal stem cells is administered between immediately after occurrence of the vascular trauma and about 7 days after occurrence of the vascular trauma; optionally wherein the therapeutic amount of mesenchymal stem cells is administered between about 3 and about 5 days after occurrence of the vascular trauma.

3. The use of claim 1, wherein a therapeutic amount is about 1 x 10⁴ to about 3 x 10⁶ mesenchymal stem cells.

4. The use of claim 1, wherein said mesenchymal stem cells are autologous or allogenic.

5. Use of mesenchymal stem cells excluding human embryonic mesenchymal stem cells in the manufacture of a medicament for diminishing restenosis in an angioplasty patient at the site of local injury to the wall of the blood vessel.

6. The use of claim 5, wherein a therapeutic amount of mesenchymal stem cells is administered in conjunction with an inflammatory response at the site of the angioplasty.

7. The use of claim 5, wherein a therapeutic amount of mesenchymal stem cells is administered between immediately after the angioplasty and about 14 days after the angioplasty; optionally wherein the therapeutic amount of mesenchymal stem cells is administered between immediately after the angioplasty and about 7 days after the angioplasty; optionally wherein the therapeutic amount of mesenchymal stem cells is administered between about 3 and about 5 days after the angioplasty.

8. The use of claim 5, wherein a therapeutic amount is about 1 × 10⁴ to about 3 x 10⁶ of mesenchymal stem cells.

9. The use of claim 5, wherein said mesenchymal stem cells are autologous or allogenic.

10. The use of claim 1 or 5, wherein said mesenchymal stem cells are administered intravenously.

## Patentansprüche

1. Verwendung von mesenchymalen Stammzellen außer menschlichen embryonalen mesenchymalen Stammzellen bei der Herstellung eines Medikaments zur Reduzierung der Restenose eines Blutgefäßes eines Patienten, der ein vaskuläres Trauma erlitten hat, wobei das vaskuläre Trauma durch einen Operationsvorgang bewirkt wird, z.B. ausgewählt aus der Gruppe bestehend aus Angioplastie, Stentimplantation, Rotoblation, Atherektomie, Thrombektomie und Transplantation.

2. Verwendung nach Anspruch 1, wobei (i) eine therapeutische Menge von mesenchymalen Stammzellen in Verbindung mit einer Entzündungsreaktion am Ort des vaskulären Traumas verabreicht wird, oder wobei (ii) eine therapeutische Menge von mesenchymalen Stammzellen zwischen direkt nach dem Auftreten des vaskulären Traumas und etwa 14 Tagen nach dem Auftreten des vaskulären Traumas verabreicht wird, wobei optional die therapeutische Menge von mesenchymalen Stammzellen zwischen direkt nach dem Auftreten des vaskulären Traumas und etwa 7 Tagen nach dem Auftreten des vaskulären Traumas verabreicht wird, wobei optional die therapeutische Menge von mesenchymalen Stammzellen zwischen etwa 3 und etwa 5 Tagen nach dem Auftreten des vaskulären Traumas verabreicht wird.

3. Verwendung nach Anspruch 1, wobei eine therapeutische Menge etwa 1x10⁴ bis 3x10⁶ mesenchymale Stammzellen ist.

4. Verwendung nach Anspruch 1, wobei die mesenchymalen Stammzellen autolog oder allogen sind.

5. Verwendung von mesenchymalen Stammzellen außer menschlichen embryonalen mesenchymalen Stammzellen bei der Herstellung eines Medikaments zur Reduzierung der Restenose eines Angioplastie-Patienten am Ort einer lokalen Verletzung der Wand des Blutgefäßes.

6. Verwendung nach Anspruch 5, wobei eine therapeutische Menge von mesenchymalen Stammzellen in Verbindung mit einer Entzündungsreaktion am Ort der Angioplastie verabreicht wird.

7. Verwendung nach Anspruch 5, wobei eine therapeutische Menge von mesenchymalen Stammzellen zwischen direkt nach der Angioplastie und etwa 14 Tagen nach der Angioplastie verabreicht wird, wobei optional die therapeutische Menge von mesenchymalen Stammzellen zwischen direkt nach der Angioplastie und etwa 7 Tagen nach der Angioplastie verabreicht wird, wobei optional die therapeutische Menge von mesenchymalen Stammzellen zwischen etwa 3 und etwa 5 Tagen nach der Angioplastie verabreicht wird.

8. Verwendung nach Anspruch 5, wobei eine therapeutische Menge etwa 1x10⁴ bis 3x10⁶ mesenchymale Stammzellen ist.

9. Verwendung nach Anspruch 5, wobei die mesenchymalen Stammzellen autolog oder allogen sind.

10. Verwendung nach Anspruch 1 oder 5, wobei die mesenchymalen Stammzellen intravenös verabreicht werden.

## Revendications

1. Utilisation de cellules souches mésenchymateuses à l'exception de cellules souches mésenchymateuses embryonnaires humaines pour la fabrication d'un médicament pour la réduction de la resténose d'un vaisseau sanguin d'un patient, qui souffre d'un traumatisme vasculaire, le traumatisme vasculaire étant causé par une intervention chirurgicale, p.ex. choisie à partir du groupe comprenant l'angioplastie, l'implantation d'un stent, la rotoblation, l'athérectomie, la thrombectomie et la transplantation.

2. Utilisation selon la revendication 1, dans laquelle (i) une quantité thérapeutique de cellules souches mésenchymateuses est administrée conjointement avec une réaction inflammatoire au site du traumatisme vasculaire, ou dans laquelle (ii) une quantité thérapeutique de cellules souches mésenchymateuses est administrée entre directement après l'occurrence du traumatisme vasculaire et environ 14 jours après l'occurrence du traumatisme vasculaire, dans laquelle optionnellement la quantité thérapeutique de cellules souches mésenchymateuses est administrée entre directement après l'occurrence du traumatisme vasculaire et environ 7 jours après l'occurrence du traumatisme vasculaire, dans laquelle optionnellement la quantité thérapeutique de cellules souches mésenchymateuses est administrée entre environ 3 et environ 5 jours après l'occurrence du traumatisme vasculaire.

3. Utilisation selon la revendication 1, dans laquelle une quantité thérapeutique est environ 1x10⁴ à 3x10⁶ cellules souches mésenchymateuses.

4. Utilisation selon la revendication 1, dans laquelle les cellules souches mésenchymateuses sont autologues ou allogéniques.

5. Utilisation de cellules souches mésenchymateuses à l'exception de cellules souches mésenchymateuses embryonnaires humaines pour la fabrication d'un médicament pour la réduction de la resténose dans un patient de l'angioplastie au site d'une lésion locale de la paroi du vaisseau sanguin.

6. Utilisation selon la revendication 5, dans laquelle une quantité thérapeutique de cellules souches mésenchymateuses est administrée conjointement avec une réaction inflammatoire au site de l'angioplastie.

7. Utilisation selon la revendication 5, dans laquelle une quantité thérapeutique de cellules souches mésenchymateuses est administrée entre directement après l'angioplastie et environ 14 jours après l'angioplastie, dans laquelle optionnellement la quantité thérapeutique de cellules souches mésenchymateuses est administrée entre directement après l'angioplastie et environ 7 jours après l'angioplastie, dans laquelle optionnellement la quantité thérapeutique de cellules souches mésenchymateuses est administrée entre environ 3 et environ 5 jours après l'angioplastie.

8. Utilisation selon la revendication 5, dans laquelle une quantité thérapeutique est environ 1x10⁴ à 3x10⁶ cellules souches mésenchymateuses.

9. Utilisation selon la revendication 5, dans laquelle les cellules souches mésenchymateuses sont autologues ou allogéniques.

10. Utilisation selon la revendication 1 ou 5, dans laquelle les cellules souches mésenchymateuses sont administrées par voie intraveineuse.
